# EUROPEAN PATENT APPLICATION

(11) **EP 4 846 922 A1**
(43) Date of publication of application: **02.12.2026**
(21) Application number: 25216820.8
(22) Date of filing: 19.11.2025
(51) Int. Cl.: A61B 3/00, A61B 3/117, A61B 3/135

(54) **IMAGING APPARATUS FOR QUANTITATIVELY OBSERVING DEGREE OF CATARACT PROGRESSION USING AUTOFLUORESCENCE**

(30) Priority: 27.05.2025 KR 20250068712
(71) Applicant: Korea University Research and Business Foundation, Seoul 02841 (KR)
(72) Inventor: EOM, Youngsub, 08011 Seoul (KR)
(74) Representative: IPAZ

(57) **Abstract**

The present specification provides an imaging apparatus capable of creating an image that may assist in quantitatively evaluating an opacity degree or a cataract grade of an examined eye. The imaging apparatus according to the present specification includes a filter having at least one scattering region having a surface treated to scatter light emitted from a light source. An autofluorescence phenomenon may occur due to the directly emitted light when the light passes through a scattering region of the filter, and an autofluorescence phenomenon may additionally occur due to the scattered light. An image sensor may generate image data having brightness proportional to the amount of charge accumulated in each pixel for an irradiation time. In this case, the created image may be an image in which the region corresponding to the scattering region in the retina is higher in brightness than the region corresponding to a background region in the retina.

## Description

### CROSS-REFERENCE TO RELATED APPLICATIONS

This application claims the priority of Korean Patent Application No. 10-2025-0068712 filed on May 27, 2025, in the Korean Intellectual Property Office, the disclosure of which is incorporated herein by reference.

### BACKGROUND

### Field

The present disclosure relates to an autofluorescence imaging apparatus, and more particularly, to an imaging apparatus for quantitatively observing a degree of cataract progression using autofluorescence.

### Description of the Related Art

The contents disclosed in this section only provide background information in respect to the embodiment disclosed in the present specification but do not necessarily constitute the prior art.

In the related art, there is a lens opacities classification system (LOCS) as a standard for classifying nuclear cataracts. The LOCS divides a lens into three parts, i.e., a cortex, a nucleus, and a posterior capsule (posterior lens capsule). The cortex and the posterior capsule are classified into five stages in accordance with opacity, and the nucleus is classified into six stages in accordance with opacity and coloration. Clinicians determine nuclear cataracts based on the LOCS on the basis of a result of directly observing a cross-section of the lens by using a slit-lamp microscope. A surgery for removing a cataract is performed by removing an opaque lens and inserting an artificial lens.

However, even if a cataract surgery may not always be necessary in accordance with a degree of cataract progression, since the current degree of cataract progression is determined only by direct observation of the clinicians, there is a problem in that a determination result may vary depending on the clinicians, and there is a likelihood that even a patient, who does not require surgery, may undergo unnecessary cataract removal surgery. Therefore, there is a need for a method capable of quantitatively determining a degree of cataract progression or a degree of presbyopia progression.

### [Document of Related Art]

### [Patent Document]

(Patent Document 1) Korean Patent Application Laid-Open No. 10-2023-0085805

### SUMMARY

An object of the present specification is to provide an imaging apparatus capable of creating an image that may assist in quantitatively evaluating an opacity degree or a cataract grade of an examined eye.

The present specification is not limited to the aforementioned technical problems, and other technical problems, which are not mentioned above, may be clearly understood by those skilled in the art from the following descriptions.

In order to achieve the above-mentioned object, the present embodiment provides an imaging apparatus including: a light source configured to irradiate an examined eye with light through a preset optical path; an image sensor configured to capture an image of the examined eye at least partially irradiated by an autofluorescence phenomenon by the light emitted from the light source; and a filter disposed between the image sensor and the examined eye. In this case, the light source may emit light so that the light projected onto the examined eye moves from one end to the other end of the examined eye for a preset irradiation time, the image sensor may generate image data having brightness proportional to the amount of charge accumulated in each pixel for the irradiation time, and the filter may have at least one scattering region having a surface treated to scatter the light emitted from the light source.

According to the embodiment of the present specification, the light emitted from the light source may have a wavelength that induces the autofluorescence phenomenon in a lens protein of the examined eye.

According to the embodiment of the present specification, the light source may emit light so that the light projected onto the examined eye has a shape of a linear light source.

According to the embodiment of the present specification, when a direction in which the light projected onto the examined eye moves from one end to the other end of the examined eye is defined as a first direction, the light source may emit light so that the light projected onto the examined eye has a shape of a point light source, and the light projected onto the examined eye further moves in a second direction orthogonal to the first direction.

According to the embodiment of the present specification, the image sensor may generate image data by light projected onto a retina of the examined eye.

According to the embodiment of the present specification, the image sensor may generate monochrome image data.

According to the embodiment of the present specification, the scattering region may be formed by a plurality of figures spaced apart from one another.

According to the embodiment of the present specification, the plurality of figures may be disposed to be horizontally symmetric or vertically symmetric with respect to a central portion.

According to the embodiment of the present specification, at least two figures, among the plurality of figures, may have different areas.

According to the embodiment of the present specification, among the figures having different areas, an area of the figure adjacent to a peripheral portion may be larger than an area of the figure adjacent to a central region.

The imaging apparatus according to the present specification may further include: a hardware processor; and a memory configured to store the image data generated by the image sensor. In this case, the memory may further store a computer program that outputs information related to an opacity degree or a cataract grade of the examined eye by using a brightness value (hereinafter, referred to as a first brightness value') of a region corresponding to the scattering region in the stored image and a brightness value (hereinafter, referred to as a 'second brightness value') of a region corresponding to a region that is not the scattering region.

According to the embodiment of the present specification, the computer program may output information related to the opacity degree or the cataract grade of the examined eye based on a level difference between the second brightness value and the first brightness value.

According to the embodiment of the present specification, the computer program may output a high opacity degree or a high cataract grade of the examined eye as the level difference between the second brightness value and the first brightness value increases.

Other detailed matters of the present disclosure are included in the detailed description and the drawings.

According to one aspect of the present specification, it is possible to capture an autofluorescence image that enables quantitative determination of the presence or absence of abnormalities of the lens. It is possible to quantitatively evaluate the presence or absence of abnormalities of the lens, which include the degree of presbyopia progression, the degree of cataract progression, or the like, based on the autofluorescence image.

According to another aspect of the present specification, it is possible to quantitatively evaluate the presence or absence of abnormalities of lenses of patients and prevent unnecessary cataract surgeries, thereby reducing patient discomfort and unnecessary surgical costs.

The effects of the present disclosure are not limited to the aforementioned effects, and other effects, which are not mentioned above, will be clearly understood by those skilled in the art from the following description.

The effects of the present disclosure are not limited to the aforementioned effects, and other effects, which are not mentioned above, will be apparently understood to a person having ordinary skill in the art from the following description.

The objects to be achieved by the present disclosure, the means for achieving the objects, and the effects of the present disclosure described above do not specify essential features of the claims, and, thus, the scope of the claims is not limited to the disclosure of the present disclosure.

### BRIEF DESCRIPTION OF DRAWINGS

The above and other aspects, features and other advantages of the present disclosure will be more clearly understood from the following detailed description taken in conjunction with the accompanying drawings, in which:
FIG. 1 is a block diagram of an imaging apparatus according to the present specification;
FIG. 2 is a reference view of the imaging apparatus according to the embodiment of the present specification;
FIG. 3 is a reference view of a process of capturing an image of an examined eye by using the imaging apparatus according to the present specification;
FIG. 4 is a reference view illustrating a simplified optical path during the process in which the imaging apparatus according to the present specification captures an image of the examined eye;
FIG. 5 is a reference view illustrating a state in which a light source of the imaging apparatus according to the present specification irradiates the examined eye with light;
FIG. 6 is an exemplified view of a filter according to the embodiment of the present specification;
FIG. 7 is a view illustrating an example in which the imaging apparatus according to the present specification captures images of lenses with different opacity degrees;
FIG. 8 is a reference view illustrating a light path in case that a general optical filter is used when no cataract exists;
FIG. 9 is a reference view illustrating a light path in case that the general optical filter is used when a cataract exists;
FIG. 10 is a reference view illustrating a light path in case that a filter according to the present specification is used when no cataract exists; and
FIGS. 11 and 12 are reference views illustrating a light path in case that the filter according to the present specification is used when a cataract exists.

### DETAILED DESCRIPTION OF THE EMBODIMENT

Hereinafter, the exemplary embodiment of the present disclosure will be described with reference to the accompanying drawings and exemplary embodiments as follows. Scales of components illustrated in the accompanying drawings are different from the real scales for the purpose of description, so that the scales are not limited to those illustrated in the drawings.

Advantages and features disclosed in the present specification and methods of achieving the advantages and features will be clear with reference to embodiments described in detail below together with the accompanying drawings. However, the present specification is not limited to the exemplary embodiments disclosed herein but will be implemented in various forms. The exemplary embodiments of the present specification are provided so that the present specification is completely disclosed, and a person with ordinary skill in the art can fully understand the scope of the present specification. The protection scope of the present specification will be defined only by the scope of the appended claims.

The terms used in the present specification are for explaining the exemplary embodiments, not for limiting the protection scope of the present specification. Unless particularly stated otherwise in the present specification, a singular form also includes a plural form. The term "comprise" and/or "comprising" used in the specification does not exclude existence or addition of one or more other constituent elements in addition to the mentioned constituent element.

Like reference denotations refer to like elements throughout the specification. As used herein, the term "and/or" includes each and all combinations of one or more of the mentioned components. Terms "first", "second", and the like may be used to describe various constituent elements, but the constituent elements are of course not limited by these terms. These terms are merely used to distinguish one constituent element from another constituent element. Therefore, the first constituent element mentioned hereinafter may of course be the second constituent element within the technical spirit of the present disclosure.

Unless otherwise defined, all terms (including technical and scientific terms) used in the present specification may be used as the meaning which may be commonly understood by the person with ordinary skill in the art to which the present specification pertains. In addition, terms defined in a generally used dictionary shall not be construed in ideal or excessively formal meanings unless they are clearly and specially defined in the present specification.

There is a fundus autofluorescence (FAF) imaging technology. Unlike fluorescein angiography, the FAF imaging does not require injection of fluorescent dye to visualize a retina. The FAF imaging creates images by utilizing fluorescent properties of lipofuscin present in a retinal pigment epithelium (RPE). In FAF images, an abnormal pattern of autofluorescence (AF) serves as a biomarker of retinal disease. Therefore, the FAF imaging in the related art has been used to evaluate retinal diseases or abnormalities, but the FAF imaging has not been used to evaluate the presence or absence of abnormalities of lenses.

Meanwhile, the inventors of the present application have discovered that when a filter including a plurality of portions with different optical transmittances is disposed between an autofluorescence imaging apparatus and an eyeball a subject (hereinafter, referred to as "examined eye") and an autofluorescence image is captured, the brightness of the image changes depending on a degree of cataract progression of the examined eye. Based on this phenomenon, the present inventors conceived an idea for quantitatively observing the degree of cataract progression, and accordingly filed Korean Patent Application No. 10-2022-0026607 with the Korean Intellectual Property Office (KIPO) on March 2, 2022.

However, Korean Patent Application No. 10-2022-0026607 merely discloses the discovered phenomenon without explaining specific optical principles. Subsequently, through further research, the present inventors discovered the optical characteristics of the autofluorescence imaging apparatus and the filter that occur due to the above-mentioned phenomenon. Therefore, in the present specification, an imaging apparatus using newly identified properties of the autofluorescence imaging apparatus and the filter will be described. For reference, the entire disclosure of Korean Patent Application No. 10-2022-0026607 may be incorporated in the present specification by reference.

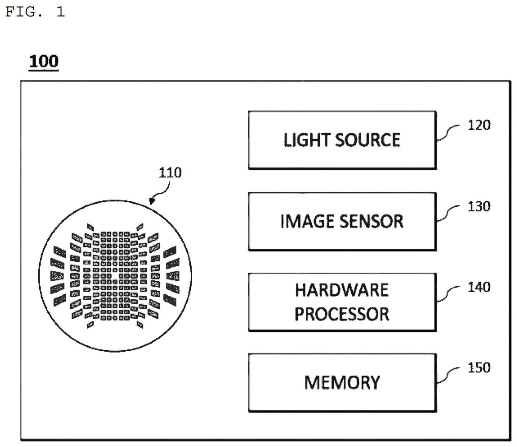

FIG. 1 is a block diagram of an imaging apparatus according to the present specification.

With reference to FIG. 1, an imaging apparatus 100 according to an embodiment of the present specification may include a filter 110, a light source 120, and an image sensor 130. The light source 120 may irradiate an examined eye with light through a preset optical path. The image sensor 130 may capture an image of the examined eye at least partially irradiated with the light emitted from the light source 120 by an autofluorescence phenomenon. The filter 110 may be disposed between the image sensor 130 and the examined eye.

The imaging apparatus 100 according to the present specification may further include a hardware processor 140 and a memory 150. Among the constituent elements of the imaging apparatus 100 according to the present specification, the light source 120, the image sensor 130, the hardware processor 140, and the memory 150 are electronic components configured to be operated by electricity. Among the constituent elements of the imaging apparatus 100 according to the present specification, the filter 110, the light source 120, and the image sensor 130 may be optical components that are affected by light or affect light.

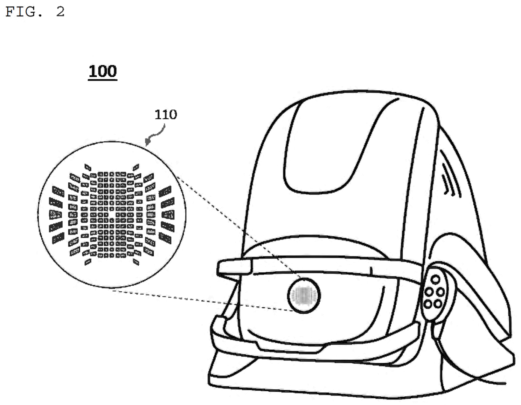

FIG. 2 is a reference view of the imaging apparatus according to the embodiment of the present specification.

With reference to FIG. 2, an example of an external shape of the imaging apparatus according to the embodiment of the present specification may be identified. Among the constituent elements of the imaging apparatus 100 according to the present specification, the light source 120, the image sensor 130, the hardware processor 140, and the memory 150 may be present in the apparatus and covered by an outer housing without being directly visible to the naked eye. Among the constituent elements of the imaging apparatus 100 according to the present specification, the filter 110 may be disposed outside the apparatus such that the presence of the filter 110 may be visibly identifiable to the naked eye.

An example of the imaging apparatus illustrated in FIG. 2 is a drawing illustrating an external shape of a product manufactured by Optos, for reference. The present inventors utilized the product manufactured by Optos during a process of discovering a phenomenon, which constitutes a key of the present disclosure, and developing the phenomenon into the invention. During this process, the present inventors recognized that the characteristics of the light source and the image sensor included in the product manufactured by Optos were related to the phenomenon that constitutes the key of the present disclosure. Therefore, the present inventors propose the product manufactured by Optos in order to enable those skilled in the art to easily understand and reproduce the present disclosure. However, the present disclosure is not necessarily limited to the product manufactured by Optos or the light source and the image sensor included in the product manufactured by Optos.

First, a process in which an autofluorescence image is captured by the light source 120, the image sensor 130, the hardware processor 140, and the memory 150 among the constituent elements of the imaging apparatus 100 according to the present specification will be described.

Meanwhile, the hardware processor may execute various control logics related to an image capturing process to be described below. The hardware processor may include a general-purpose microprocessor, an application-specific integrated circuit (ASIC), other chipsets, logic circuits, registers, communication modems, data processing devices, and the like. In addition, the hardware processor 140 may execute a computer program stored in the memory.

The memory 150 may refer to a device-readable medium which stores data on a semipermanent basis rather than a medium, such as a register or a cache, which stores data during a short moment. In addition, the memory may store a computer program related to the image capturing process to be described below.

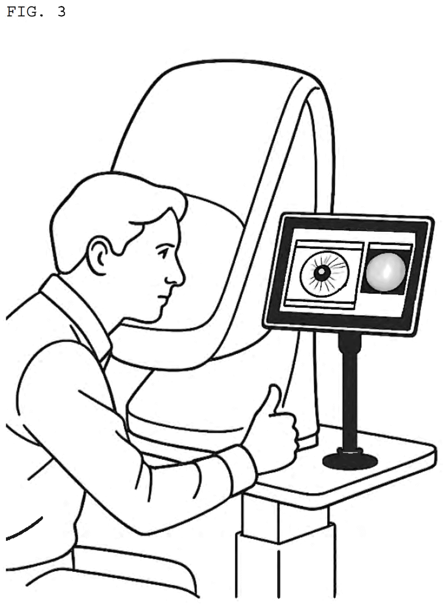

FIG. 3 is a reference view of a process of capturing an image of the examined eye by using the imaging apparatus according to the present specification.

As illustrated in FIG. 3, the image capturing process is performed in a state in which one eyeball of the subject is tightly attached to the imaging apparatus 100.

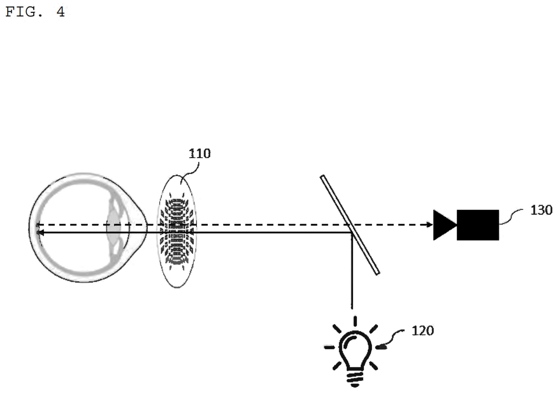

FIG. 4 is a reference view illustrating a simplified optical path during the process in which the imaging apparatus according to the present specification captures an image of the examined eye.

The light emitted from the light source 120 may have a wavelength capable of inducing the autofluorescence phenomenon in a lens protein of the examined eye. A main protein, which induces the autofluorescence in the cataract in the lens, is a crystallin family protein. The cataract occurs due to denaturation or aggregation of the lens protein. In particular, α-, β-, and γ-crystallins are modified by aging or oxidative stress. The light source 120 may emit light having a wavelength range (of about 320 to 370 nm) in which the crystallin family protein is excited.

The image sensor 130 may generate image data from the light projected onto the retina of the examined eye. In particular, it is apparent that optical elements, such as lenses, may be used to allow the image sensor 130 to capture an image of the retina. According to the embodiment of the present specification, the image sensor may generate monochrome image data. The term "monochrome image (black-and-white image) refers to an image that includes only brightness (intensity, luminance) information for each pixel, i.e., an image that includes only a degree of contrast (lightness and darkness) without color (hue) or chroma (saturation) information. For example, the monochrome image may be a grayscale image represented by 256 levels of brightness.

Because the basic optical principle and configuration of the apparatus for capturing an autofluorescence image is a publicly-known technology, a detailed description thereof will be omitted. However, according to the present specification, the light source 120 and the image sensor 130 of the imaging apparatus 100 needs to have the following characteristics.

First, the light source 120 may emit light so as to scan the examined eye or the filter 110. In this case, the light source 120 may emit light so that the light passes each region of the examined eye or each region of the filter 110 only once. In other words, the light source 120 may emit light such that the light does not repetitively reach the same region of the examined eye or the filter 110.

To this end, in the imaging apparatus 100 according to the present specification, the light source 120 may emit light so that the light, which is projected onto the examined eye, moves from one end to the other end of the examined eye for a preset irradiation time. The irradiation time may be variously set, for example, to 1 seconds, 2 seconds, 3 seconds, 5 seconds, or the like.

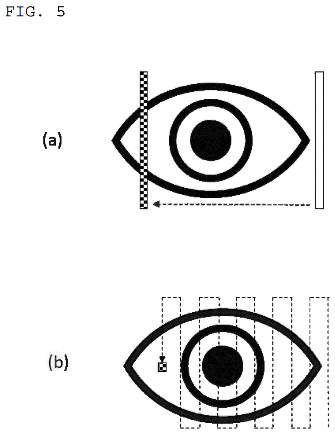

FIG. 5 is a reference view illustrating a state in which the light source of the imaging apparatus according to the present specification irradiates the examined eye with light.

With reference to FIG. 5A, the light source 120 may emit light so that the light projected onto the examined eye has a shape of a linear light source. The term "linear light source" refers to light having a length significantly larger than a width thereof to the extent that the linear light source appears as a single line. A direction in which the light projected onto the examined eye moves from one end to the other end of the examined eye is defined as a first direction. In the example illustrated in FIG. 5, the first direction is illustrated as a direction in which the light moves from the right side to the left side.

With reference to FIG. 5B, the light source 120 may emit light so that the light projected onto the examined eye has a shape of a point light source. In this case, the light source 120 may emit light so that the light projected onto the examined eye further moves in a second direction orthogonal to the first direction. In the example illustrated in FIG. 5, the second direction is illustrated as an upward-downward direction/downward-upward direction.

Next, the image sensor 130 may generate image data having brightness proportional to the amount of charge accumulated in each pixel for the irradiation time. The image sensor 130 may include a charge-coupled device (CCD). The CCD may be operated based on a photoelectric effect (photogeneration) in which incident light (photons) generates electron-hole pairs inside a semiconductor, and the charge (electrons) generated in each pixel is accumulated (charge accumulation). Simply, the CCD is a sensor that accumulates electrons generated by incident light in each pixel. Therefore, the image sensor 130 maintains a shutter or aperture in an open state for the irradiation time so that each pixel may accumulate light.

Lastly, the filter 110 included in the imaging apparatus 100 according to the present specification may define at least one scattering region having a surface treated to scatter the light emitted from the light source 120.

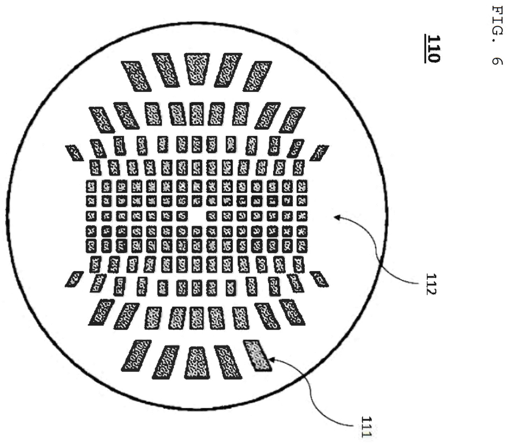

FIG. 6 is an exemplified view of the filter according to the embodiment of the present specification.

With reference to FIG. 6, a scattering region 111 and a background region 112 may be identified. The filter 110 may have a glass or plastic material. The scattering region 111 may be implemented in a form that changes surface roughness. For example, the scattering region 111 may be implemented in a frosted glass state by rubbing or corroding glass. In another example, the scattering region 111 may be implemented as etched glass by applying a corrosion liquid or laser processing onto glass surface or implemented as sandblasted glass by pneumatically blasting sand or emery particles. The background region 112 refers to a region of the filter 110 that has not been processed separately. Therefore, the background region 112 is a region in which a scattering phenomenon occurs remarkably less when the light passes through the filter in comparison with the scattering region 111.

The scattering region 111 may be formed by a plurality of figures spaced apart from one another. The plurality of figures may be disposed to be horizontally symmetric or vertically symmetric with respect to a central portion. Further, among the plurality of figures, at least two figures may have different areas. In this case, among the figures having different areas, an area of the figure adjacent to a peripheral portion is larger than an area of the figure adjacent to a central region. Various embodiments of the filter 110 and the shape of the scattering region 111 may be incorporated by reference from the disclosure of Korean Patent Application No. 10-2022-0026607.

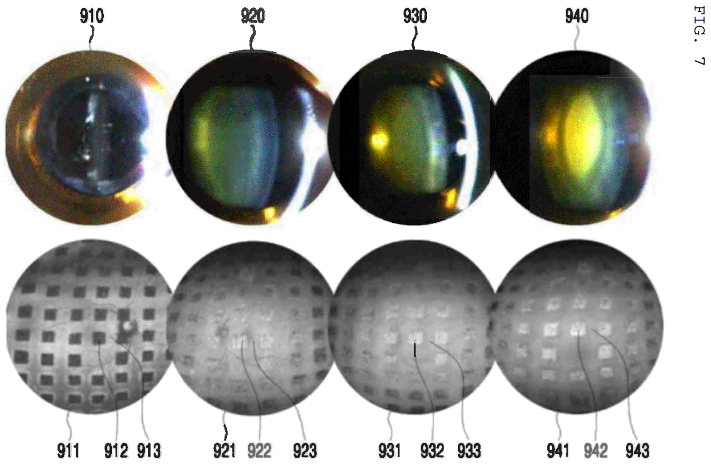

When the imaging apparatus 100 according to the present specification captures an image of the eyeball of the cataract patient, an image such as that illustrated in in FIG. 7 may be obtained.

FIG. 7 is a view illustrating an example in which the imaging apparatus according to the present specification captures images of lenses with different opacity degrees.

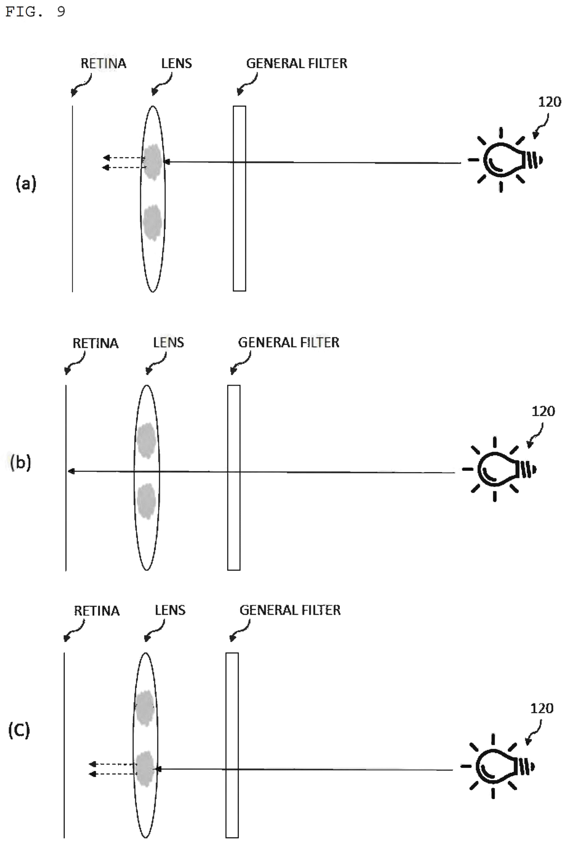

With reference to FIG. 7, contrary to the general expectation that the autofluorescence of the retina will be blocked by the opacity of the lens, the present inventors identified a phenomenon in which the brightness of the region corresponding to the scattering region in the image was increased by the autofluorescence of the lens. Further, it can be ascertained that the brightness of the region corresponding to the scattering region in the image increases as the degree of cataract progression or the degree of presbyopia progression related to the opacity of the lens increases. The present inventors conceived an idea for quantitatively evaluating the degree of cataract progression or the degree of presbyopia progression by the above-mentioned phenomenon. For reference, the image illustrated in FIG. 7 is an image illustrated in FIG. 9 of Korean Patent Application No. 10-2022-0026607, and the reference numerals are maintained in an intact manner for convenience. Therefore, the specific description of the image illustrated in FIG. 7 may be referred to in the disclosure related to FIG. 9 of Korean Patent Application No. 10-2022-0026607, and the specific description will be omitted in the present specification.

However, as disclosed in Korean Patent Application No. 10-2022-0026607, when the present inventors discovered the above phenomenon, the present inventors only hypothesized the optical principle and did not provide a detailed explanation thereof. In the present specification, it will be explained how the autofluorescence of the lens causes an increase in brightness in the region corresponding to the scattering region within the image contrary to the general expectation that the autofluorescence of the retinal will be blocked by the opacity of the lens.

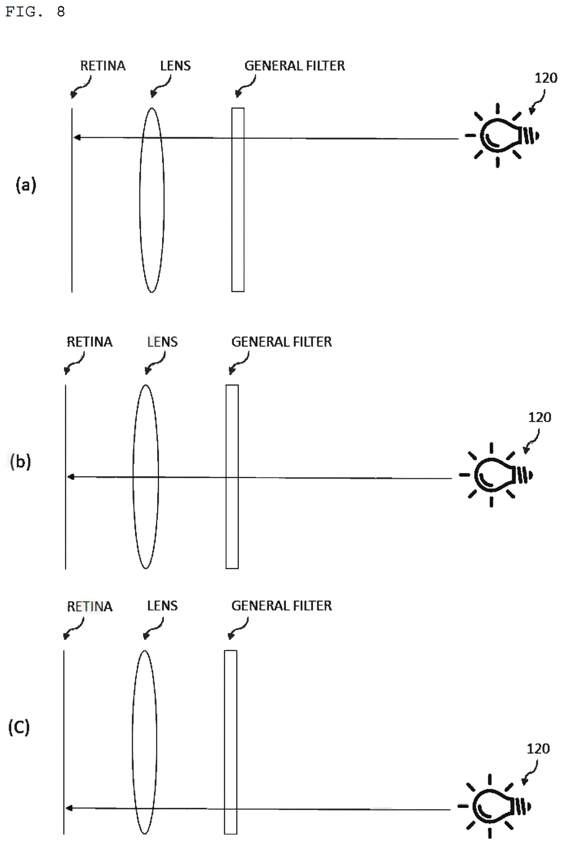

FIGS. 8 to 12 schematically illustrate a process in which the light emitted from the light source 120 of the imaging apparatus 100 according to the present specification is projected onto the retina via the optical filter and the lens of the examined eye. The image sensor 130 generates image data by capturing an image of the light projected onto the retina. The following description will describe how the light obtained by the autofluorescence occurring in the lens is projected onto the retina.

FIG. 8 is a reference view illustrating a light path in case that a general optical filter is used when no cataract exists.

With reference to FIGS. 8A to 8C, it is assumed that the lens of the subject is in a clear state in which no cataract occurs. Further, the filter used in FIGS. 8A to 8C is a general optical filter. The term "general optical filter" refers to a filter that does not have characteristics of the filter 110 included in the imaging apparatus 100 according to the present specification. More specifically, the filter 110 included in the imaging apparatus 100 according to the present specification has the scattering region 111, but a general filter does not have a separate scattering region. Therefore, very fine scattering may occur when light passes through the general filter. However, the scattering may be ignored because the scattering is small to the extent that the scattering does not affect the image. The light source 120 included in the imaging apparatus 100 according to the present specification may emit light so that the light moves from one end to the other end of the examined eye for the preset irradiation time. Therefore, FIGS. 8A, 8B, and 8C sequentially illustrate the movement of light with which the examined eye is irradiated for the irradiation time.

The image sensor 130 may generate image data having brightness proportional to the amount of charge accumulated in each pixel for a period of time corresponding to FIGS. 8A to 8C. In this case, the created image may be an image of a retina of a normal person.

FIG. 9 is a reference view illustrating a light path in case that the general optical filter is used when a cataract exists.

When FIG. 9 is compared with FIG. 8, it can be ascertained that a figure, which indicates the occurrence of cataract, is added within the lens. With reference to FIG. 9A, when the light emitted from the light source 120 passes through the general filter and reaches a cataract region, an autofluorescence phenomenon occurs in the corresponding region. Further, the light (dotted arrow) emitted by the autofluorescence may be projected onto the retina. With reference to FIG. 9B, in the region having no cataract, the light emitted from the light source 120 passes through the general filter and the lens and reaches the retina. With reference to FIG. 9C, when the light emitted from the light source 120 passes through the general filter and reaches another cataract region, an autofluorescence phenomenon occurs in the corresponding region. Further, the light (dotted arrow) emitted by the autofluorescence may be projected onto the retina.

The image sensor 130 may generate image data having brightness proportional to the amount of charge accumulated in each pixel for a period of time corresponding to FIGS. 9A to 9C. In this case, the created image may be an image different from an image of a region different in brightness from a region corresponding to the region in which cataract occurs in the lens in the retina region. In this case, the corresponding region may be brighter due to the autofluorescence or be darker by blocking a part of the light emitted from the light source 120.

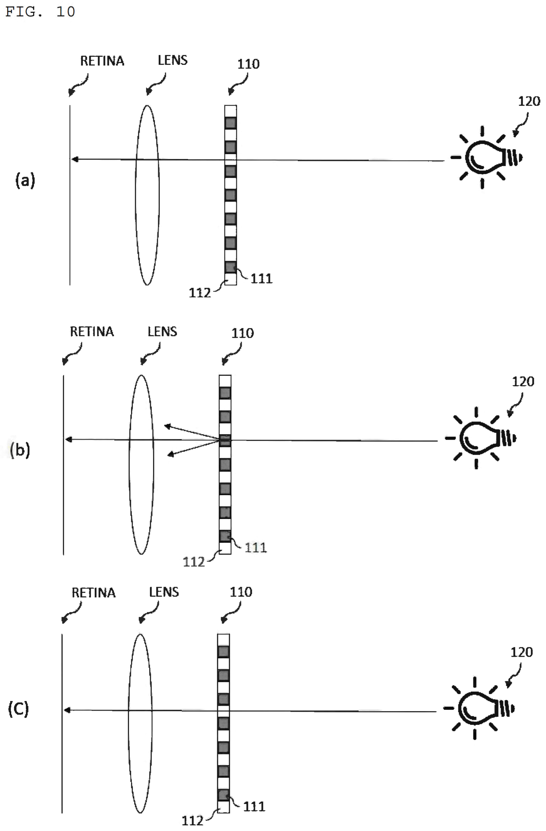

FIG. 10 is a reference view illustrating a light path in case that a filter according to the present specification is used when no cataract exists.

When FIG. 10 is compared with FIGS. 8 and 9, it can be ascertained that the lens of the subject is in the clear state in which no cataract occurs, and the scattering region 111 and the background region 112 of the filter 110 repeatedly define a pattern. FIGS. 10A and 10C illustrate situations in which the light emitted from the light source 120 passes through the background region 112 of the filter 110. In this case, because no light scattering occurs, the light emitted from the light source 120 reaches the retina almost in an intact manner. FIG. 10B illustrates a situation in which the light emitted from the light source passes through the scattering region 111 of the filter 110. Because the scattering region 111 of the filter 110 is relatively lower in light transmittance than the background region 112, the intensity of the light reaching the retina is relatively lower than the light having passed through the background region 112. In addition, FIG. 10B illustrates that the light emitted from the light source 120 scatters while passing through the scattering region 111 of the filter 110.

The image sensor 130 may generate image data having brightness proportional to the amount of charge accumulated in each pixel for a period of time corresponding to FIGS. 10A to 10C. In this case, the created image may be an image including a repetitive pattern of the scattering region 111 and the background region 112 of the filter 110 in the retina region (reference numeral 911 in FIG. 7). The light scattered in the scattering region 111 of the filter 110 may pass through the lens and reach the retina. However, because the scattered light uniformly reaches the region corresponding to the scattering region 111 and the background region 112 in the region of the retina, the region (reference numeral 912 in FIG. 7) corresponding to the scattering region in the retina is lower in brightness than the region (reference numeral 913 in FIG. 7) corresponding to the background region in the retina.

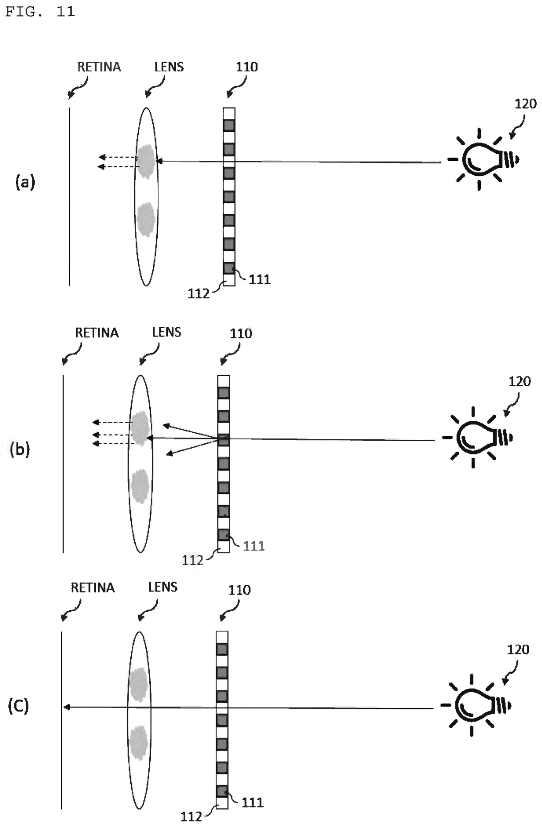

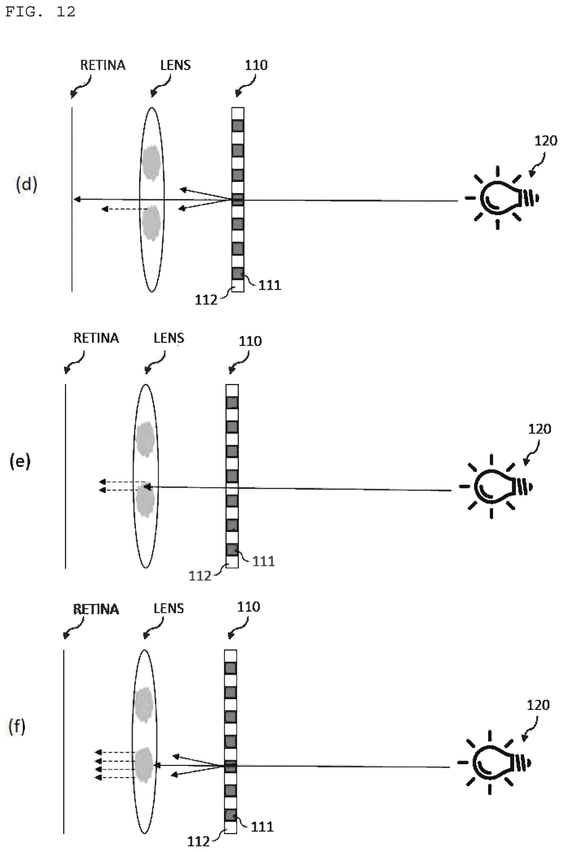

FIGS. 11 and 12 are reference views illustrating a light path in case that the filter according to the present specification is used when a cataract exists.

FIG. 11A illustrates a situation in which the light emitted from the light source 120 passes through the background region 112 of the filter 110 and reaches the region in which a cataract occurs at an upper side in the lens. In the corresponding region, an autofluorescence phenomenon may occur, and the light (dotted arrow) emitted by the autofluorescence may be projected onto the retina.

FIG. 11B illustrates a situation in which the light emitted from the light source 120 passes through the scattering region 111 of the filter 110 and reaches the region in which a cataract occurs at an upper side in the lens. It can be ascertained that in the corresponding region, the autofluorescence phenomenon occurs due to the directly emitted light, and the autofluorescence phenomenon also occurs additionally due to the scattered light. The region in which the autofluorescence phenomenon additionally occurs due to the scattered light may overlap a region illustrated in FIG. 11A. In other words, in the region in which the cataract occurs in the lens, the autofluorescence phenomenon may occur once due to the light emitted directly from the light source 120, and the autofluorescence phenomenon may occur once more due to the light scattered in the scattering region 111. In other words, in the region in which the cataract occurs in the lens, the autofluorescence phenomenon may occur once due to the light emitted directly from the light source 120, and the autofluorescence phenomenon may be further maintained due to the light scattered in the scattering region 111. Therefore, the amount of charge accumulated in each pixel for the irradiation time may increase.

FIG. 11C illustrates a situation in which the light emitted from the light source 120 passes through the background region 112 of the filter 110, passes through the region in which no cataract occurs in the lens, and reaches the retina.

FIG. 12D illustrates a situation in which the light emitted from the light source 120 passes through the scattering region 111 of the filter 110 and reaches the region in which no cataract occurs in the lens. It can be ascertained that even though the autofluorescence phenomenon does not occur due to the light emitted directly from the light source 120, the autofluorescence phenomenon occurs due to the scattered light in the region in which a cataract occurs at a lower side in the lens.

FIG. 12E illustrates a situation in which the light emitted from the light source 120 passes through the background region 112 of the filter 110 and reaches the region in which a cataract occurs at the lower side in the lens. It can be ascertained that the autofluorescence phenomenon occurs in the corresponding region.

FIG. 12F illustrates a situation in which the light emitted from the light source 120 passes through the scattering region 111 of the filter 110 and reaches the region in which a cataract occurs at the lower side in the lens. It can be ascertained that in the corresponding region, the autofluorescence phenomenon occurs due to the directly emitted light, and the autofluorescence phenomenon also occurs additionally due to the scattered light.

With reference to FIGS. 12D to 12F, it can be ascertained that in the region in which the cataract occurs at the lower side in the lens, the autofluorescence phenomenon occurs due to the scattered light before the light emitted from the light source 120 directly reaches the region, the autofluorescence phenomenon occurs even when the light emitted from the light source 120 directly reaches the region, and the autofluorescence phenomenon occurs due to the scattered light even after the light emitted from the light source 120 passes through the region.

The image sensor 130 may generate image data having brightness proportional to the amount of charge accumulated in each pixel for a period of time corresponding to FIGS. 11 and 12A to 12F. In this case, the created image may be an image including a repetitive pattern of the scattering region 111 and the background region 112 of the filter 110 in the retina region. However, in this case, the created image may be an image in which the region (reference numeral 942 in FIG. 7) corresponding to the scattering region in the retina is higher in brightness than the region (reference numeral 943 in FIG. 7) corresponding to the background region in the retina.

In the image data generated by the image sensor 130, the brightness of the region corresponding to the scattering region in the image increases as the degree of opacity or the degree of cataract progression of the examined eye increases. With the use of these characteristics, it is possible to quantitatively calculate the opacity degree or the cataract grade of the examined eye.

According to the embodiment of the present specification, the memory 150 may store the image data generated by the image sensor. Further, in the stored image, the memory 150 may further store a computer program that outputs information related to the opacity degree or the cataract grade of the examined eye by using a brightness value (hereinafter, referred to as a first brightness value') of the region corresponding to the scattering region and a brightness value (hereinafter, referred to as a 'second brightness value') of the region corresponding to a region that is not the scattering region. The hardware processor may further execute the computer program.

The computer program may output information related to the opacity degree or the cataract grade of the examined eye on the basis of a level difference between the second brightness value and the first brightness value. More specifically, the computer program may output a high opacity degree or a high cataract grade of the examined eye as the level difference between the second brightness value and the first brightness value increases.

In order for the computer to read the program and execute the methods implemented with the program, the computer program may include a code encoded into a computer language such as C/C++, C#, Java, Python, or a machine language readable through a device interface of the computer by a processor (CPU) of the computer. Such a code may include a functional code associated with a function and the like defining functions necessary for executing the methods and may include a control code associated with an execution procedure necessary for the processor of the computer to execute the functions according to a predetermined procedure. Further, such a code may further include a code associated with memory reference about whether additional information or media necessary for the processor of the computer to execute the functions is referred at any location (address number) of an internal or external memory of the computer. Further, if it is necessary for the processor of the computer to communicate with any computer or server located in a remote place to execute the functions, the code may further include a communication related code about how communication is performed with any computer or server located in a remote place using a communication module of the computer and whether to transmit and receive any information or media upon communication.

While the embodiments of the present specification have been described above with reference to the accompanying drawings, those skilled in the art will understand that the present specification may be carried out in any other specific form without changing the technical spirit or an essential feature thereof. Therefore, it should be understood that the above-described embodiments are illustrative in all aspects and do not limit the present disclosure.

## Claims

1. An imaging apparatus comprising:
a light source configured to irradiate an examined eye with light through a preset optical path;
an image sensor configured to capture an image of the examined eye at least partially irradiated by an autofluorescence phenomenon by the light emitted from the light source; and
a filter disposed between the image sensor and the examined eye,
wherein the light source emits light so that the light projected onto the examined eye moves from one end to the other end of the examined eye for a preset irradiation time,
wherein the image sensor generates image data having brightness proportional to the amount of charge accumulated in each pixel for the irradiation time, and
wherein the filter has at least one scattering region having a surface treated to scatter the light emitted from the light source.

2. The imaging apparatus of claim 1, wherein the light emitted from the light source has a wavelength that induces the autofluorescence phenomenon in a lens protein of the examined eye.

3. The imaging apparatus of claim 1, wherein the light source emits light so that the light projected onto the examined eye has a shape of a linear light source.

4. The imaging apparatus of claim 1, wherein when a direction in which the light projected onto the examined eye moves from one end to the other end of the examined eye is defined as a first direction, the light source emits light so that the light projected onto the examined eye has a shape of a point light source, and the light projected onto the examined eye further moves in a second direction orthogonal to the first direction.

5. The imaging apparatus of claim 1, wherein the image sensor generates image data by light projected onto a retina of the examined eye.

6. The imaging apparatus of claim 1, wherein the image sensor generates monochrome image data.

7. The imaging apparatus of claim 1, wherein the scattering region is formed by a plurality of figures spaced apart from one another.

8. The imaging apparatus of claim 7, wherein the plurality of figures are disposed to be horizontally symmetric or vertically symmetric with respect to a central portion.

9. The imaging apparatus of claim 7, wherein at least two figures, among the plurality of figures, have different areas.

10. The imaging apparatus of claim 9, wherein among the figures having different areas, an area of the figure adjacent to a peripheral portion is larger than an area of the figure adjacent to a central region.

11. The imaging apparatus of claim 1, further comprising:
a hardware processor; and
a memory configured to store the image data generated by the image sensor,
wherein the memory further stores a computer program that outputs information related to an opacity degree or a cataract grade of the examined eye by using a brightness value (hereinafter, referred to as a first brightness value') of a region corresponding to the scattering region in the stored image and a brightness value (hereinafter, referred to as a 'second brightness value') of a region corresponding to a region that is not the scattering region.

12. The imaging apparatus of claim 11, wherein the computer program outputs information related to the opacity degree or the cataract grade of the examined eye based on a level difference between the second brightness value and the first brightness value.

13. The imaging apparatus of claim 12, wherein the computer program outputs a high opacity degree or a high cataract grade of the examined eye as the level difference between the second brightness value and the first brightness value increases.

## Amended claims

### Amended claims in accordance with Rule 137(2) EPC.

1. An imaging apparatus (100) comprising:
a light source (120) configured to irradiate an examined eye with light through a preset optical path;
an image sensor (130) configured to capture an image of the examined eye at least partially irradiated by an autofluorescence phenomenon by the light emitted from the light source (120); and
a filter (110) disposed between the image sensor (130) and the examined eye,
wherein the light source (120) emits light so that the light projected onto the examined eye moves from one end to the other end of the examined eye for a preset irradiation time,
wherein the image sensor (130) generates image data having brightness proportional to the amount of charge accumulated in each pixel for the irradiation time, and
wherein the filter (110) has at least one scattering region (111) having a surface treated to scatter the light emitted from the light source (120).

2. The imaging apparatus (100) of claim 1, wherein the light emitted from the light source (120) has a wavelength that induces the autofluorescence phenomenon in a lens protein of the examined eye.

3. The imaging apparatus (100) of claim 1, wherein the light source (120) emits light so that the light projected onto the examined eye has a shape of a linear light source.

4. The imaging apparatus (100) of claim 1, wherein when a direction in which the light projected onto the examined eye moves from one end to the other end of the examined eye is defined as a first direction, the light source (120) emits light so that the light projected onto the examined eye has a shape of a point light source, and the light projected onto the examined eye further moves in a second direction orthogonal to the first direction.

5. The imaging apparatus (100) of claim 1, wherein the image sensor (130) generates image data by light projected onto a retina of the examined eye.

6. The imaging apparatus (100) of claim 1, wherein the image sensor (130) generates monochrome image data.

7. The imaging apparatus (100) of claim 1, wherein the scattering region (111) is formed by a plurality of figures spaced apart from one another.

8. The imaging apparatus (100) of claim 7, wherein the plurality of figures are disposed to be horizontally symmetric or vertically symmetric with respect to a central portion.

9. The imaging apparatus (100) of claim 7, wherein at least two figures, among the plurality of figures, have different areas.

10. The imaging apparatus (100) of claim 9, wherein among the figures having different areas, an area of the figure adjacent to a peripheral portion is larger than an area of the figure adjacent to a central region.

11. The imaging apparatus (100) of claim 1, further comprising:
a hardware processor (140); and
a memory (150) configured to store the image data generated by the image sensor (130),
wherein the memory (150) further stores a computer program that outputs information related to an opacity degree or a cataract grade of the examined eye by using a brightness value (hereinafter, referred to as a 'first brightness value') of a region corresponding to the scattering region (111) in the stored image and a brightness value (hereinafter, referred to as a 'second brightness value') of a region corresponding to a region that is not the scattering region (111).

12. The imaging apparatus (100) of claim 11, wherein the computer program outputs information related to the opacity degree or the cataract grade of the examined eye based on a level difference between the second brightness value and the first brightness value.

13. The imaging apparatus (100) of claim 12, wherein the computer program outputs a high opacity degree or a high cataract grade of the examined eye as the level difference between the second brightness value and the first brightness value increases.
